# EUROPEAN PATENT SPECIFICATION

(11) **EP 3 165 597 B1**
(45) Date of publication and mention of the grant of the patent: **06.03.2019**
(21) Application number: 16196011.7
(22) Date of filing: 27.10.2016
(51) Int. Cl.: C12M 1/00, C12M 1/34

(54) **OBSERVATION APPARATUS**
BEOBACHTUNGSVORRICHTUNG
APPAREIL D'OBSERVATION

(30) Priority: 05.11.2015 JP 2015217775
(43) Date of publication of application: 10.05.2017
(73) Proprietor: Olympus Corporation, Hachioji-shi Tokyo 192-8507 (JP)
(72) Inventor: UE, Yoshihiro, Tokyo 192-8507 (JP); ENDO, Tomio, Tokyo 192-8507 (JP); ONO, Osamu, Tokyo 192-8507 (JP); MATSUSHITA, Akira, Tokyo 192-8507 (JP); TANIKAWA, Yohei, Tokyo 192-8507 (JP)
(74) Representative: Gunzelmann, Rainer

(56) References cited:
- EP-A1- 1 672 405
- EP-A1- 1 916 296
- EP-A1- 2 272 971
- EP-A2- 2 199 380
- US-A1- 2005 105 172

## Description

### FIELD

The embodiments disclosed herein are related to an observation apparatus placed in an incubator. The scope of protection is defined by the appended claims.

### BACKGROUND

Conventionally, in order to maintain a prescribed culture environment, the culturing of a biological sample such as a cell has been performed in an incubator. During the culturing, the state of growth of the biological sample is regularly checked. At this time, when the biological sample is taken out of the incubator, the temperature of the biological sample may decrease, and it may negatively affect the growth of the biological sample. For this reason, an observation apparatus has been proposed for observing a biological sample that is being cultured without taking it out of the incubator (for example, see Japanese Laid-open Patent Publication No. 2012-003036) . EP 2 199 380 A2 discloses an cell culture observation unit to be used in an incubator where cells are cultured.

However, when observing a biological sample without taking it out of the incubator, heat generated in the observation apparatus transfers to the biological sample, and the temperature of the biological sample may increases in some cases. An increase in the temperature of the biological sample, as with a decrease in the temperature, is undesirable as it may negatively affect the growth of the biological sample.

In view of the situation above, an objective of the present invention is to provide a technique for observing a biological sample that is being cultured while suppressing heat transfer to the sample.

### SUMMARY

One embodiment of the present invention provides a system with an observation apparatus to be placed in an incubator, equipped with a housing including a top plate that is a stage on which a container that accommodates a sample is placed; an optical system accommodated in the housing; an imaging device configured to capture an image of the sample with light from the sample that has entered through the stage and the optical system; and a motor that is a power source of a moving mechanism configured to make the optical system move in a direction along the stage, where at least one of the imaging device or the motor is placed outside the housing.

According to the present invention, a technique for observing a biological sample that is being cultured while suppressing heat transfer in the sample may be provided.

### BRIEF DESCRIPTION OF DRAWINGS

The present invention will be more apparent from the following detailed description when the accompanying drawings are referenced.
FIG. 1 is a sectional view of an observation apparatus 100 according to Embodiment 1 not forming part of the present invention.
FIG. 2 is a plan view of the observation apparatus 100 illustrated in FIG. 1.
FIG. 3 is a diagram that illustrates an example of a fixing unit 80 that fixes the observation apparatus 100 illustrated in FIG. 1 in an incubator 200.
FIG. 4 is a flowchart of an observation method using the observation apparatus 100 illustrated in FIG. 1
FIG. 5 is a sectional view of an observation apparatus 300 according to Embodiment 2 not forming part of the present invention.
FIG. 6 is a plan view of the observation apparatus 300 illustrated in FIG. 5.
FIG. 7 is a sectional view of an observation apparatus 400 according to Embodiment 3 not forming part of the present invention.
FIG. 8 is a plan view of the observation apparatus 400 illustrated in FIG. 7.
FIG. 9 is a sectional view of an observation apparatus 500 according to Embodiment 4, which is an embodiment according to the present invention.
FIG. 10 is a plan view of the observation apparatus 500 illustrated in FIG. 9.
FIG. 11 is a sectional view of an observation apparatus 600 according to Embodiment 5, which is an embodiment according to the present invention.
FIG. 12 is a plan view of the observation apparatus 600 illustrated in FIG. 11.

### DESCRIPTION OF EMBODIMENTS

### <Embodiment 1>

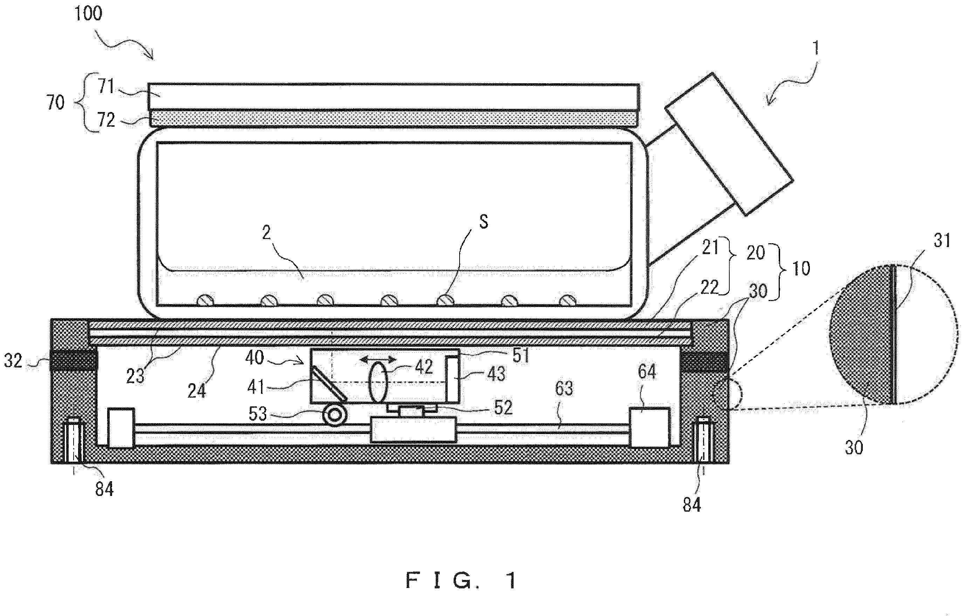

FIG. 1 is a sectional view of an observation apparatus 100 according to the present embodiment. FIG, 2 is a plan view of the observation apparatus 100. The observation apparatus 100 is an observation apparatus for observing a biological sample that is being cultured in an incubator without taking it out of the incubator. The observation apparatus 100 is placed in the incubator.

The observation apparatus 100 is equipped with a housing 10 that includes a stage 20 and a frame 30, as illustrated in FIG. 1. A container 1 that accommodates a biological sample S is placed on the stage 20. The container 1 is an optically transparent container that accommodates the biological sample S. The biological sample S is cultured in a state immersed in a culture solution 2 in the container 1.

The stage 20 is the top plate of the housing 10. The stage 20 is configured to form, together with the frame 30, a sealed internal space in the housing 10. In addition, the stage 20 is configured so that the thermal resistance of the stage
20 is higher than the thermal resistance of the frame 30. Specifically, the stage 20 is equipped with two flat plates (a flat plate 21, a flat plate 22) which is placed so that a vacuum is sandwiched between the two flat plates. Here, vacuum refers to a state in which the pressure is lower than atmospheric pressure. The interposition of a vacuum makes it possible to suppress heat conduction and conductive heat transfer, and to increase thermal resistance.

The flat plate 21 and the flat plate 22 are glass plates, for example. The material of the flat plates that constitute the stage 20 is not limited to glass as long as it is optically transparent. An antireflection films 23 are formed on the surface of the flat plate 21 and the surface of the flat plate 22 by a surface treatment. In addition, a surface treatment for reflecting infrared rays is also applied to the flat plate 22, so as to suppress the passage of radiant heat generated inside the housing 10 through the stage 20. Accordingly, a heat ray reflecting film 24 is also formed on the surface of the flat plate 22. Meanwhile, it is desirable that the heat ray reflecting film 24 be formed, on the flat plate 22, on its surface that closer to the internal space.

The frame 30 is configured so as to form, together with the stage 20, a sealed inner space in the housing 10. It is preferable that the material of the frame 30 be a material having a high thermal conductivity, such as aluminum, for example. After a cutting process, no particular surface treatment is applied to the inner surface of the frame 30. On the other hand, a surface treatment for facilitating heat dissipation from the inner space to outside the housing 10 is applied to the outer surface of the frame 30. Specifically, a heat dissipation paint 31 is applied to the outer surface of the frame 30. Furthermore, a screw hole into which a screw 84 of a fixing unit 80 explained later is inserted is formed at four positions of the bottom surface of the frame 30.

The housing 10 is further equipped with a heat insulation sheet 32 that is a heat insulating member that blocks heat conduction from the frame 30 to the stage 20. Meanwhile, FIG. 1 illustrates an example in which the heat insulation sheet 32 is embedded in the frame 30, but the heat insulation sheet 32 may be provided between the stage 20 and the frame 30, for example.

The observation apparatus 100 is equipped with, in its housing 10, an imaging unit 40, and a moving mechanism that makes the imaging unit 40 move in the housing 10. The moving mechanism is a mechanism that makes the imaging unit 40 move two-dimensionally in a direction along the stage 20. The moving mechanism is a scanning mechanism that scans the biological sample S in the container 1.

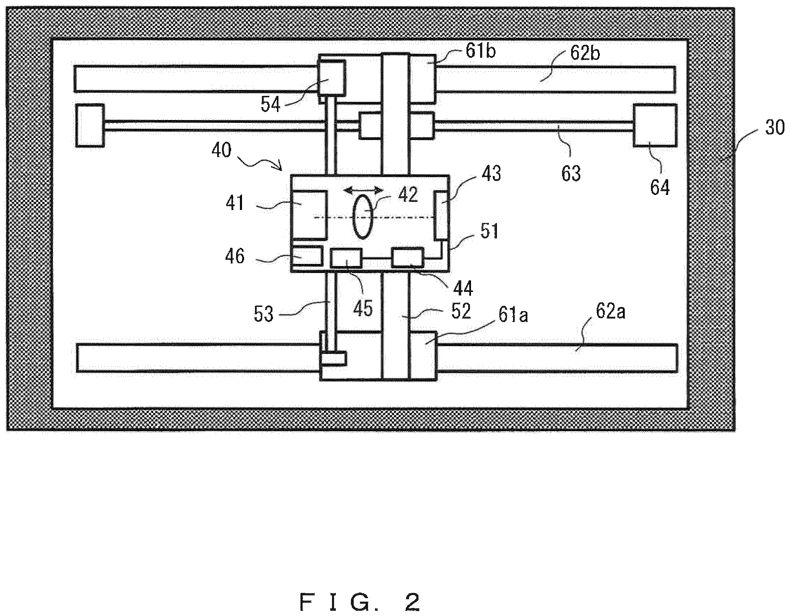

The imaging unit 40 is provided in a slider 51 that constitutes the moving mechanism. The imaging unit 40 is equipped with an optical system (a mirror 41, an objective 42), an imaging device 43, a control apparatus 44, a transmission/reception apparatus 45, and a light source apparatus 46, as illustrated in FIG. 2.

The mirror 41 is a deflecting member that directs the light from the biological sample S entering the imaging unit 40 through the stage 20 in the direction of the optical axis of the objective 42. The objective 42 is an optical system accommodated in the housing 10. The objective 42 is placed with its optical axis directed in the direction along the stage 20. The objective 42 is formed with one or more lenses including a lens that move in the direction of the optical axis (hereinafter, referred to as a moving group) . The objective 42 is configured so that the focal length of the objective 42 is changed by the movement of the moving group.

The imaging device 43 is a CCD (Charge-Coupled Device) image sensor, or a CMOS (Complementary MOS) image sensor, for example. The imaging device 43 is accommodated in the housing 10. The imaging device 43 captures the image of the biological sample S with the light from the biological sample S that has entered through the stage 20, the mirror 41, and the objective 42.

The control apparatus 44 is an apparatus that controls the observation apparatus 100. The control apparatus 44 controls the moving mechanism according an instruction received at the transmission/reception apparatus 45, for example. Accordingly, the field of view of the observation apparatus 100 may be changed from outside the observation apparatus 100. The transmission/reception apparatus 45 is a transmission apparatus that transmits image signals output from the imaging device 43 to an external device, and it is also a reception apparatus that receives instructions from an external apparatus.

The light source apparatus 46 is an LED (light emitting diode) light source, for example. The illumination light emitted from the light source apparatus 46 enters the container 1 through the stage 20, and the illumination light reflected on the top surface of the container 1 illuminates the biological sample S from above.

The moving mechanism that makes the imaging unit 40 move is controlled by the control apparatus 44. The moving mechanism is equipped with the slider 51, a guide rail 52, a ball screw 53, and a motor 54 as a mechanism for moving the imaging unit 40 linearly in one direction along the stage 20. Meanwhile, the moving mechanism is equipped with a slider 61a and a slider 61b, a guide rail 62a and a guide rail 62b, a ball screw 63, and a motor 64 as a mechanism for moving the imaging unit 40 linearly in another direction along the stage 20. The motor 54 and the motor 64 are the power source of the moving mechanism.

The slider 51 is configured to slide along the guide rail 52. The control apparatus 44 makes the motor 54 rotate, according to an instruction received at the transmission/reception apparatus 45. The rotation of the motor 54 makes the ball screw 53 rotate, and as a result, the slider 51 fixed on a nut combined with the ball screw 53 slides along the guide rail 52. Accordingly, the imaging unit 40 also moves along the guide rail 52.

The slider 61a is configured to slide along guide rail 62a, and the slider 61b is configured to slide along the guide rail 62b. Meanwhile, the guide rail 62a and the guide rail 62b are mutually parallel and directed in a direction that is orthogonal to the guide rail 52. The control apparatus 44 makes the motor 64 rotate, according to an instruction received at the transmission/reception apparatus 45. The rotation of the motor 64 makes the ball screw 63 rotate, and accordingly, the guide rail 52 fixed on a nut combined with the ball screw 63 moves. The guide rail 52 is fixed on the slider 61a and the slider 61b. Accordingly, together with the guide rail 52, the slider 61a and the slider 61b move along the guide rail 62a and the guide rail 62b. Accordingly, the imaging unit 40 also moves along the guide rail 62a and the guide rail 62b.

The observation apparatus 100 is further equipped with a heat insulation unit 70 that may be freely attached to the top surface of the container 1. The heat insulation unit 70 includes a heat insulation sheet 71 and a heat-storing gel 72. By placing the heat-storing gel 72 on the container 1 so as to be in contact with the top surface of the container 1, even when there is a temperature difference between the container 1 and outside air (atmosphere in the incubator), heat dissipation from the top surface of the container 1 is suppressed, and decreases in the temperature of the top surface of the container 1 may be suppressed.

In the observation apparatus 100 configured as described above, the biological sample S is illuminated with the illumination light from the light source apparatus 46, and an image of the biological sample S is captured with the light from the biological sample S that has entered the imaging device 43 through the objective 42. Then, by transmitting the image signal obtained at the imaging device 43 by the transmission/reception apparatus 45, the image of the biological sample S is displayed on a display apparatus placed outside the incubator. Accordingly, with the observation apparatus 100, the observer may observe the biological sample S on an external device without taking the biological sample S out of the incubator.

Meanwhile, in the observation apparatus 100, thermal resistance of the stage 20 is higher than thermal resistance of the frame 30, and therefore, a large portion of the heat generated from the heat sources (for example, the motor 54, the motor 64, the imaging device 43, the light source apparatus 46, and the like) of the observation apparatus 100 is released to outside the observation apparatus 100 through the frame 30. Accordingly, heat transfer to the biological sample S through the stage 20 is significantly suppressed. For this reason, the biological sample S may be observed while avoiding an occurrence of increases in the temperature of the biological sample S that would hinder the growth of the biological sample S.

Furthermore, in the observation apparatus 100, decreases in the temperature of the top surface of the container 1 may be suppressed by placing the heat insulation unit 70 on the top surface of the container 1. For this reason, even when a temperature difference is generated between the top surface of the container 1 and the atmosphere in the incubator, it is possible to prevent fogging of the top surface of the container 1 due to an occurrence of condensation on the top surface of the container 1. Accordingly, even when there is a temperature difference between the container 1 and the atmosphere in the incubator, observation images with a stable quality may be obtained with the observation apparatus 100.

Meanwhile, by forming a sealed inner space with the stage 20 and the housing 10, even when humidity in the incubator is high (generally, when used at 90% or above), it is possible to prevent the imaging unit 40 (the optical system, the imaging device and the like) placed in the inner space from being negatively affected due to humidity (fogging of the surface of the imaging device, the optical system, and the like). Accordingly, observation images of a stable quality may be obtained with the observation apparatus 100.

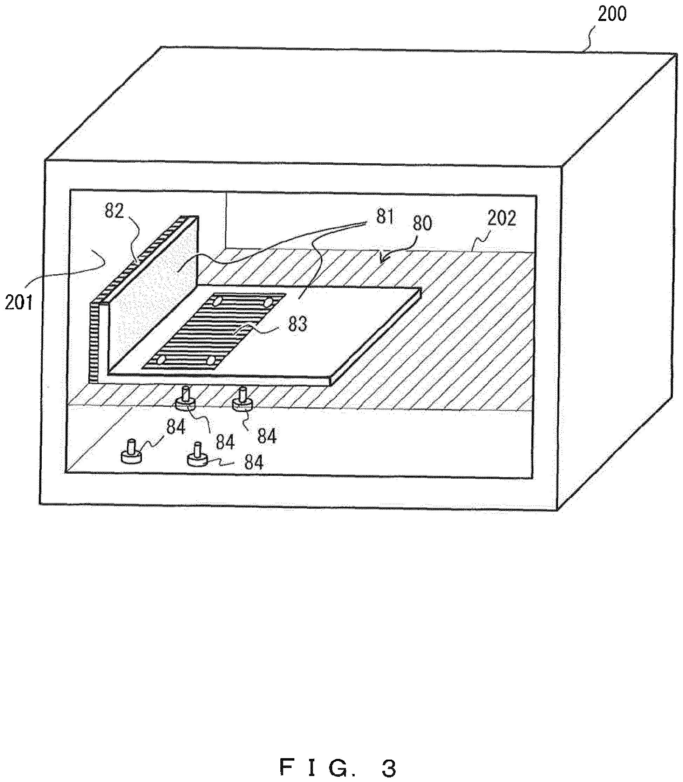

In order to further suppress increases in the temperature of the biological sample S, it is desirable that the observation apparatus 100 be equipped with a fixing unit 80 like the one illustrated in FIG. 3 and be fixed in the incubator 200 by means of the fixing unit 80. FIG. 3 is a diagram that illustrates an example of the fixing unit 80 that fixes the observation apparatus 100 presented in FIG. 1 in the incubator 200. The fixing unit 80 is explained below with reference to FIG. 3.

The fixing unit 80 is a unit that fixes the observation apparatus 100 on an inner wall 201 of the incubator 200. The fixing unit 80 is equipped with a placement stand 81 on which the housing 10 is placed, high thermal conductivity sheets (a high thermal conductivity sheet 82, a high thermal conductivity sheet 83), and a screw 84.

The placement stand 81 is an L-shaped member, made of aluminum, for example. The placement stand 81 is placed on a rack 202 installed in the incubator 200 so that an upright portion raised vertically from the bottom portion of the placement stand 81 is in contact with the inner wall of the incubator 200.

The high thermal conductivity sheet 82 is placed between the placement stand 81 and the inner wall 201, in close contact with the placement stand 81 and the inner wall 201. By providing the high thermal conductivity sheet 82, thermal resistance between the placement stand 81 and the inner wall 201 may be decreased. The high thermal conductivity sheet 83 is placed between the placement stand 81 and the housing 10, in close contact with the placement stand 81 and the housing 10. By providing the high thermal conductivity sheet 83, thermal resistance between the placement stand 81 and the housing 10 may be decreased. Meanwhile, the fixing unit 80 may be equipped with only one of either the high thermal conductivity sheet 82 or the high thermal conductivity sheet 83.

The screw 84 is inserted in a long hole formed in the placement stand 81, and screwed into a screw hole formed in the frame 30. By the screwing of the screw 84 with the frame 30, the placement stand 81 and the housing 10 are securely fixed.

By fixing the observation apparatus 100 onto the inner wall 201 of the incubator 200 by means of the fixing unit 80 as described above, the heat transferred from the heat sources of the observation apparatus 100 to the frame 30 is efficiently released to outside the observation apparatus (particularly, to the inner wall of the incubator 200 and the atmosphere in the incubator 200) through the fixing unit 80. For this reason, the heat transfer to the biological sample S through the stage 20 may be further suppressed. As a result, an occurrence of increases in the temperature of the biological sample S that would hinder the growth of the biological sample S may be avoided more certainly.

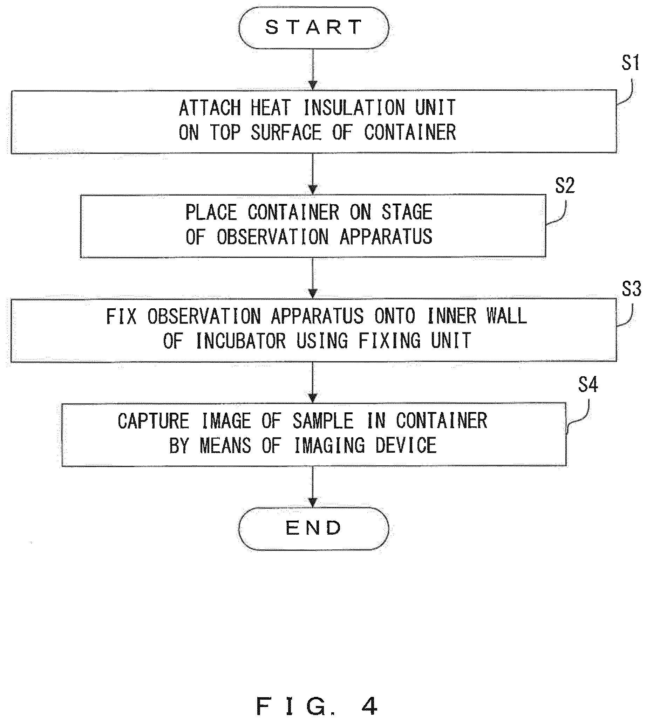

FIG. 4 is a flowchart of an observation method using the observation apparatus 100 illustrated in FIG. 1. Observation of the biological sample S by the observation apparatus 100 is performed in the procedures presented in FIG. 4, for example. First, the observer attaches the heat insulation unit 70 on the top surface of the container 1 (step S1), and places the container 1 on the stage 20 of the observation apparatus 100 (step S2). After that, the observer fixes the observation apparatus 100 onto the inner wall 201 of the incubator 200 using the fixing unit 80, to place it in the incubator 200 (step S3) . Meanwhile, step S1 through step S3 may be performed in any order. Finally, at regular intervals or at a timing desired by the observer, the imaging device 43 captures the image of the biological sample S in the container 1 (step S4). Accordingly, observation of the biological sample S may be performed without taking the biological sample S out of the incubator 200, while suppressing heat transfer from the observation apparatus 100 to the biological sample S.

### <Embodiment 2>

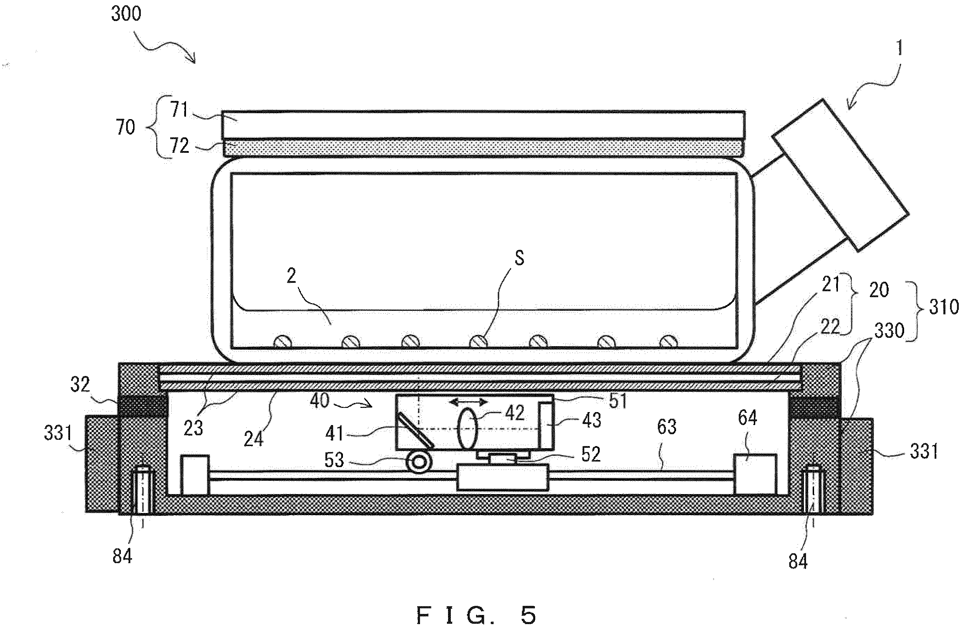

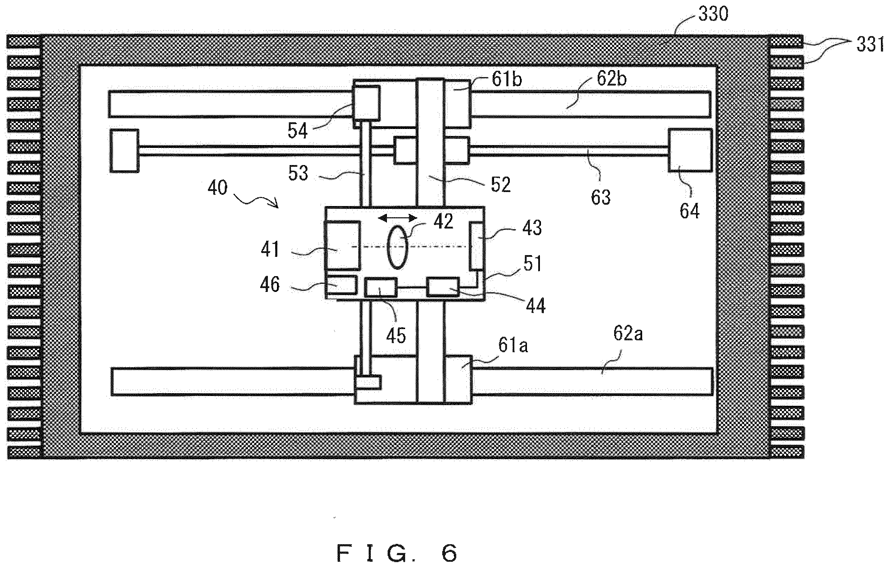

FIG. 5 is a sectional view of an observation apparatus 300 according to the present embodiment. FIG. 6 is a plan view of the observation apparatus 300. The observation apparatus 300 is an observation apparatus for observing a biological sample that is being cultured in an incubator without taking it out of the incubator. The feature of the observation apparatus 300 that it is to be placed in the incubator is similar to that of the observation apparatus 100.

The observation apparatus 300 is different from the observation apparatus 100 in that it is equipped with a housing 310 instead of the housing 10. The housing 310 is different from the housing 10 in that it is equipped with a frame 330 instead of the frame 30.

The frame 330 is configured so as to form, with the stage 20, a sealed inner space in the housing 310. Meanwhile, the frame 330 is configured so that thermal resistance of the stage 20 is higher than thermal resistance of the frame 330. The frame 330 is different from the frame 30 in that it is equipped with a fin 331 that is a projection-shaped structure that promotes heat dissipation to outside the housing 310, but the other features are similar to those of the frame 30.

With the observation apparatus 300, in the same manner as with the observation apparatus 100, the observer may observe a biological sample S on an external device without taking the biological sample S out of the incubator. Meanwhile, a large portion of the heat generated from the heat sources of the observation apparatus 300 is released to outside the observation apparatus 300 through the frame 330. For this reason, observation of the biological sample S may be performed while avoiding an occurrence of increases in the temperature of the biological sample S that would hinder the growth of the biological sample S. The features wherein an occurrence of increases in the temperature of the biological sample S may be avoided more certainly by fixing the observation apparatus 300 onto the inner wall 201 of the incubator 200 by means of the fixing unit 80, and wherein observation images with a stable quality may be obtained by placing the heat insulation unit 70 on the top surface of the container 1, are also similar.

### <Embodiment 3>

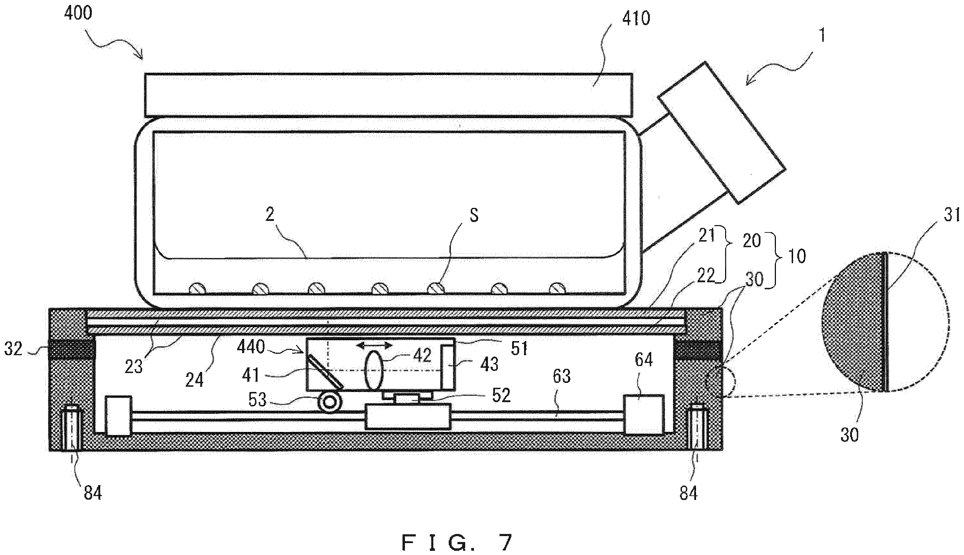

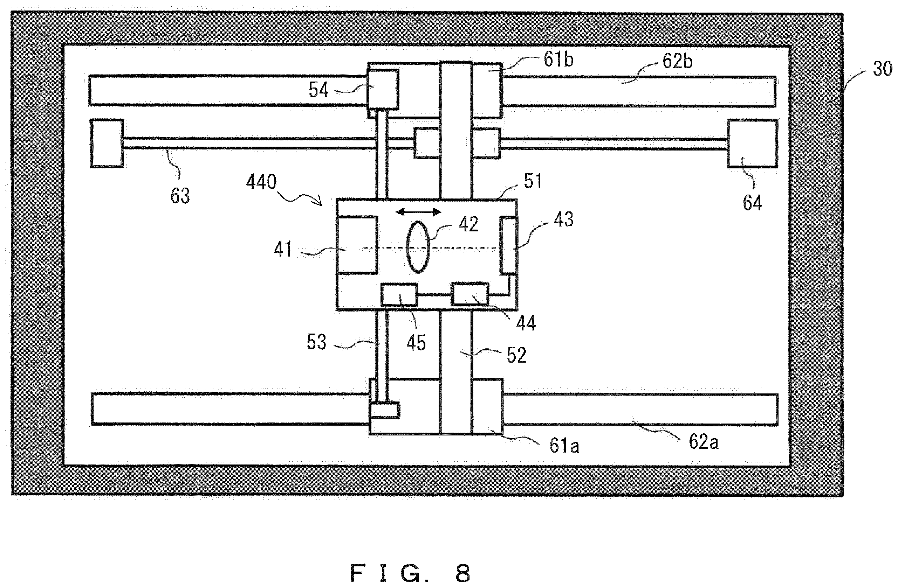

FIG. 7 is a sectional view of an observation apparatus 400 according to the present embodiment. FIG. 8 is a plan view of the observation apparatus 400. The observation apparatus 400 is an observation apparatus for observing a biological sample that is being cultured in an incubator without taking it out of the incubator. The feature of the observation apparatus 400 wherein it is to be placed in the incubator is similar to that of the observation apparatus 100.

The observation apparatus 400 is different from the observation apparatus 100 in that it is equipped with a light source apparatus 410 placed outside the housing 10 instead of the heat insulation unit 70, and with an imaging unit 440 instead of the imaging unit 40. The light source apparatus 410 is a light source apparatus that emits illumination light, and illuminates the biological sample S with illumination light. The light source apparatus 410 is configured so that it may be freely attached to the top surface of the container 1. The imaging unit 440 is different from the imaging unit 40 in that it does not include the light source apparatus 46, as illustrated in FIG. 8. The other features are similar to those of the imaging unit 40.

With the observation apparatus 400, in the same manner as with the observation apparatus 100, the observer may observe the biological sample S on an external device without taking the biological sample S out of the incubator. In addition, the features wherein the biological sample S may be observed while avoiding an occurrence of increases in the temperature of the biological sample S that would hinder the growth of the biological sample S, and wherein an occurrence of increases in the temperature of the biological sample S may be avoided more certainly by using the fixing unit 80, are also similar to those of the observation apparatus 100.

Furthermore, in the observation apparatus 400, increases in the temperature inside the housing 10 may be kept low because the light source apparatus 410 that is the main heat source is provided outside the housing 10. For this reason, heat transfer to the biological sample S through the stage 20 may be further suppressed.

Meanwhile, the light source apparatus 410 functions in a similar manner as the heat insulation unit 70. For this reason, by the installation of the light source apparatus 410 on the top surface of the container 1, fogging of the top surface of the container 1 due to an occurrence of condensation on the top surface of the container 1 may be avoided. Therefore, even when there is a temperature difference between the container 1 and the atmosphere in the incubator, observation images with a stable quality may be obtained with the observation apparatus 400.

### <Embodiment 4>

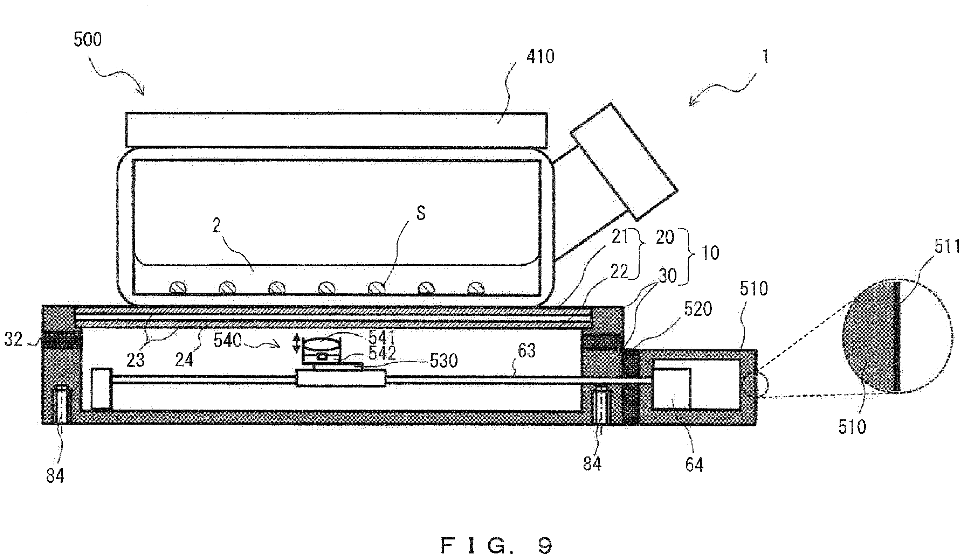

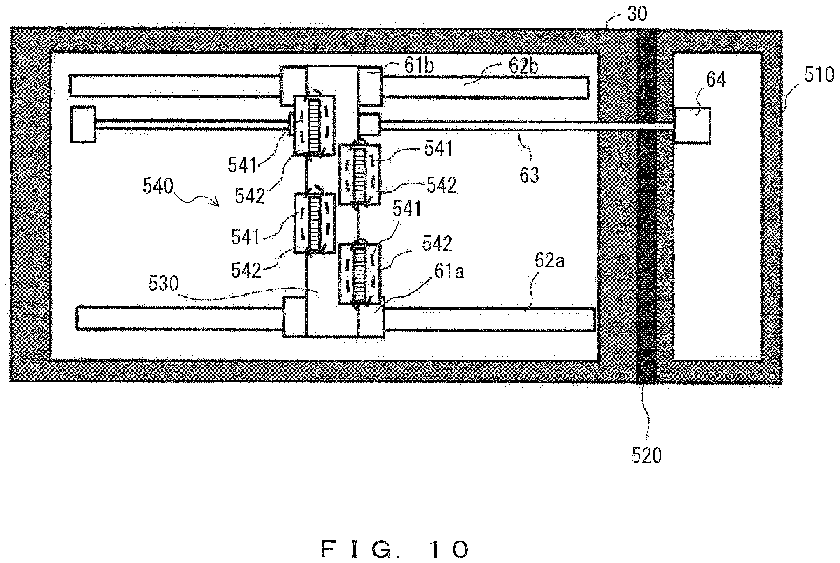

FIG. 9 is a sectional view of an observation apparatus 500 according to the present embodiment. FIG. 10 is a plan view of an observation apparatus 500. The observation apparatus 500 is an observation apparatus for observing a biological sample that is being cultured in an incubator without taking it out of the incubator. The feature of the observation apparatus 500 that it is to be placed inside the incubator is similar to that of the observation apparatus 400.

The observation apparatus 500 is equipped with, in its housing 10, an imaging unit 540 and a moving mechanism that makes the imaging unit 540 move in the housing 10. The moving mechanism is a mechanism that makes the imaging unit 540 move in a direction along the stage 20. The moving mechanism is a scanning mechanism that scans the biological sample S in the container 1. The moving mechanism of the observation apparatus 400 is a mechanism that makes the imaging unit 440 move two-dimensionally, whereas the moving mechanism of the observation apparatus 500 is a mechanism that makes the imaging unit 540 move one-dimensionally in a direction along the stage 20.

The moving mechanism that makes the imaging unit 540 move is controlled by a control apparatus that is not illustrated in the drawing. The moving mechanism is equipped with the slider 61a and the slider 61b, the guide rail 62a and the guide rail 62b, the ball screw 63, the motor 64, and a supporting member 530. The motor 64 is accommodated in a heat source accommodating unit 510 that is independent from the housing 10. That is, the motor 64 that is the power source of the moving mechanism is placed outside the housing 10.

In order to prevent the heat generated from the motor 64 from being transferred to the housing 10, a heat insulation sheet 520 is provided between the housing 10 and the heat source accommodating unit 510. The ball screw 63 penetrates through the frame 30 and the heat insulation sheet 520 and is connected to the motor 64 accommodated in the heat source accommodating unit 510.

The supporting member 530 is a member that supports the imaging unit 540 and that is fixed on a nut combined with the ball screw 63. The supporting member 530 is further fixed on the slider 61a and the slider 61b as well. Accordingly, by the rotation of the motor 64, the supporting member 530 moves along the guide rail 62a and the guide rail 62b, which makes the imaging unit 540 move along the guide rail 62a and the guide rail 62b.

The imaging unit 540 includes a plurality of imaging components. Each of the components is equipped with an objective 541 (optical system) placed with its optical axis directed in the direction that is orthogonal to the stage 20, and an imaging device 542 that captures the image of the biological sample S with the light from the biological sample S that has entered through the stage 20 and the objective 541. The imaging device 542 is a CCD line sensor or a CMOS line sensor that is equipped with a plurality of pixels arranged in the direction that is orthogonal (hereinafter, referred to as the orthogonal direction) to the moving direction of the moving mechanism, for example. The plurality of imaging components are placed so that, when the sample is scanned, at least a part of the imaging target area of each imaging component overlaps with the imaging target area of another imaging component. Meanwhile, the image signal that is output from the imaging device 542 is transmitted to an external apparatus by a transmission/reception apparatus that is not illustrated in the drawing.

With the observation apparatus 500, in the same manner as with the observation apparatus 400, the observer may observe the biological sample S on an external device without taking the biological sample S out of the incubator. In addition, the features wherein the biological sample S may be observed while avoiding an occurrence of increases in the temperature of the biological sample S that would hinder the growth of the biological sample S, and wherein an occurrence of increases in the temperature of the biological sample S may be avoided more certainly by using the fixing unit 80, are also similar to those of the observation apparatus 400. Meanwhile, in order to release heat more efficiently, it is preferable that the fixing unit 80 fix the observation apparatus 500 so that the heat source accommodating unit 510 is in contact with the upright portion of the placement stand 81. The feature wherein observation images with a stable quality may be obtained with the observation apparatus 500 because the light source apparatus 410 functions in a manner similar to the heat insulation unit 70 is similar to that in the observation apparatus 400.

In the observation apparatus 500, in addition to the light source apparatus 410 that is the main heat source, the motor 64 is also placed outside the housing 10. For this reason, according to the observation apparatus 500, increases in the temperature inside the housing 10 may be kept lower. Therefore, heat transfer to the biological sample S through the stage 20 may be further suppressed than in the observation apparatus 400.

### <Embodiment 5>

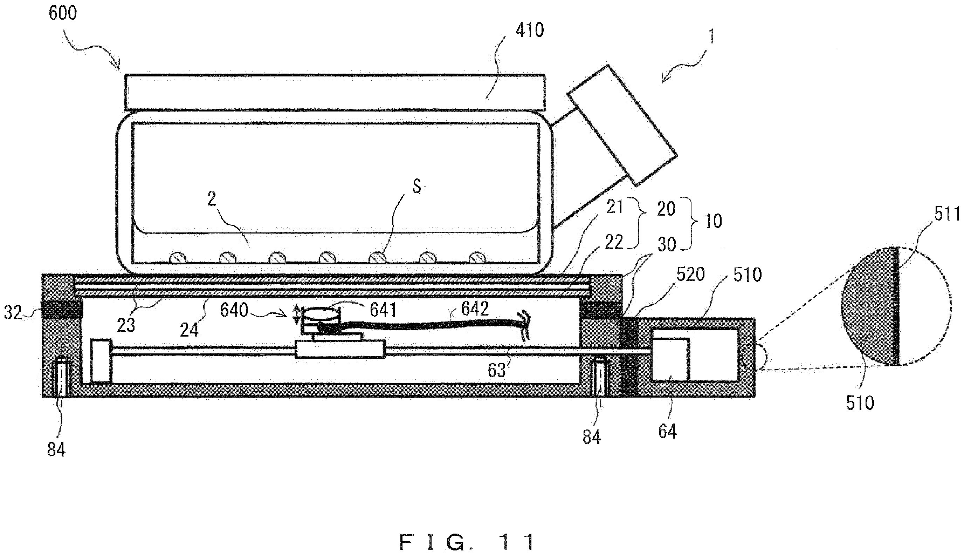

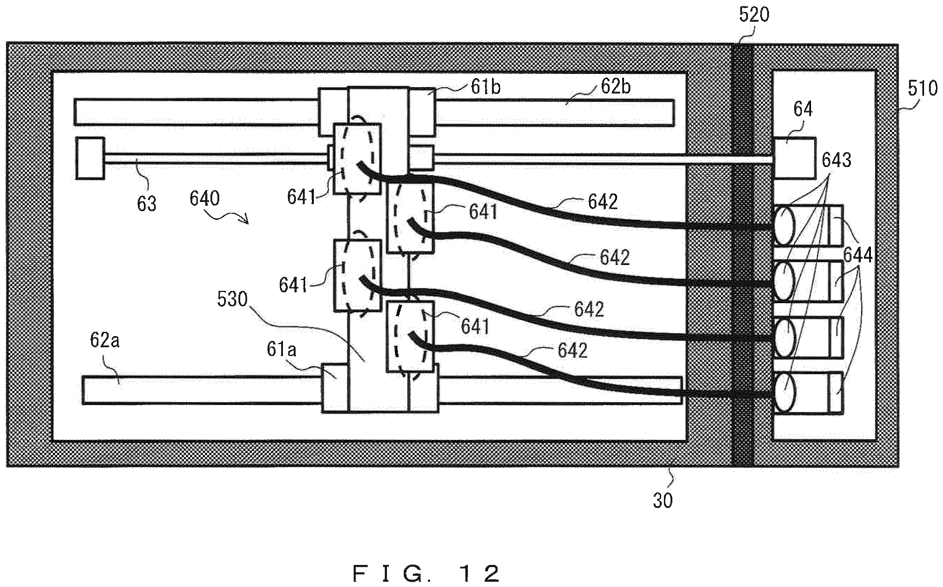

FIG. 11 is a sectional view of an observation apparatus 600 according to the present embodiment. FIG. 12 is a plan view of the observation apparatus 600. The observation apparatus 600 is an observation apparatus for observing a biological sample that is being cultured in an incubator without taking it out of the incubator. The feature of the observation apparatus 600 that it is to be placed in the incubator is similar to that of the observation apparatus 500.

The observation apparatus 600 is different from the observation apparatus 500 in that it is equipped with an imaging unit 640 instead of the imaging unit 540. The feature of the imaging unit 640 wherein it includes a plurality of imaging components is similar to that of the imaging unit 540.

Each of the imaging components of the imaging unit 640 is equipped with an objective 641, an optical fiber 642, a lens 643, and an imaging device 644. The lens 643 and the imaging device 644 are accommodated in the heat source accommodating unit 510 that is independent of the housing 10. That is, the lens 643 and the imaging device 644 are placed outside the housing 10. The optical fiber 642 is placed in the optical path between the objective 641 and the imaging device 644 placed outside the housing 10, and guides the light from the biological sample S from inside the housing 10 to outside the housing 10. More specifically, the entrance end of the optical fiber 642 is fixed on the supporting member 530, and the emitting end is fixed in the heat source accommodating unit 510. In the imaging unit 640, the light collected by the objective 641 is guided to the imaging device 644 through the optical fiber 642 and the lens 643. The image signal output from the imaging device 644 is output to an external apparatus by a transmission/reception apparatus that is not illustrated in the drawing.

With the observation apparatus 600, in the same manner as with the observation apparatus 500, the observer may observe the biological sample S on an external device without taking the biological sample S out of the incubator. In addition, the features wherein the biological sample S may be observed while avoiding an occurrence of a temperature rise of the biological sample S that would hinder the growth of the biological sample S, and wherein an occurrence of a temperature rise of the biological sample S may be avoided more certainly by using the fixing unit 80, are also similar to those of the observation apparatus 500. The feature wherein observation images with a stable quality may be obtained with the observation apparatus 600 because the light source apparatus 410 functions in a manner similar to the heat insulation unit 70 is similar to the observation apparatus 500.

In the observation apparatus 600, in addition to the light source apparatus 410 and the motor 64 that are the main heat source, the imaging device 644 is also placed outside the housing 10. For this reason, according to the observation apparatus 600, increases in the temperature inside the housing 10 may be kept lower. Therefore, heat transfer to the biological sample S through the stage 20 may be further suppressed than in the observation apparatus 500.

## Claims

1. A system comprising:
an incubator (200); and
an observation apparatus (500; 600) placed in the incubator (200), wherein
the observation apparatus (500; 600) includes
a housing (10) including a top plate that is a stage (20) on which a container (1) that accommodates a sample is placed;
an optical system (541; 641) accommodated in the housing (10);
an imaging device (542; 644) configured to capture an image of the sample with light from the sample that has entered through the stage (20) and the optical system (541; 641); and
a motor (64) that is a power source of a moving mechanism configured to make the optical system (541; 641) move in a direction along the stage (20), wherein
at least one of the imaging device (542; 644) or the motor (64) is placed outside the housing (10).

2. The system according to claim 1, the observation apparatus further comprising
an optical fiber (642) placed in an optical path between the optical system (641) and the imaging device (644) placed outside the housing (10), configured to guide the light from the sample from inside the housing (10) to outside the housing (10).

3. The system according to claim 1 or 2, wherein
both the imaging device (644) and the motor (64) are placed outside the housing (10).

4. The system according to one of claim 1 through claim 3, further comprising
a light source apparatus (410) placed outside the housing (10), configured to emit illumination light that illuminates the sample.

5. The system according to claim 4, wherein
the light source apparatus (410) is configured so as to be freely attachable to the container (1).

6. The system according to one of claim 1 through claim 5, wherein
the housing (10) includes a frame (30) configured to form an internal space with the stage (20), and
thermal resistance of the stage (20) is higher than thermal resistance of the frame (30).

7. The system according to claim 6, wherein
the stage (20) includes at least two flat plates (21;22) which is placed so that a vacuum is sandwiched between the two flat plates.

8. The system according to claim 6 or claim 7, wherein
the stage (20) includes a flat plate (22) to which a surface treatment for reflecting an infrared ray has been applied.

9. The system according to one of claim 6 through claim 8, wherein
the housing (10) includes the frame (30) to which a surface treatment for promoting heat dissipation to outside the housing (10) has been applied.

10. The system according to one of claim 6 through claim 9, wherein
the frame (30) includes a projection-shaped structure configured to promote heat dissipation to outside the housing (10).

11. The system according to one of claim 6 through claim 10, wherein
the housing (10) further includes a heat insulating member (32) configured to block heat transfer from the frame (30) to the stage (20).

12. The system according to one of claim 1 through claim 11, further comprising
a fixing unit (80) configured to fix the observation apparatus (500; 600) onto an inner wall of the incubator (200),
wherein
the fixing unit (80) includes
a placement stand (81) on which the housing (10) is placed; and
at least one of a first heat conduction member (82) between the placement stand (81) and the inner wall (201), configured to be in close contact with the placement stand (81) and the inner wall (201), or a second heat conduction member (83) between the placement stand (81) and the housing (10), configured to be in close contact with the placement stand (81) and the housing (10).

13. The system according to one of claim 1 through claim 12, further comprising
a heat insulation unit (70) configured so as to be freely attachable on a top surface of the container (1) and to suppress a decrease in a temperature of a top surface of the container (1).

14. The system according to one of claim 1 through claim 13, wherein
the housing (10) includes a frame (30) configured to form an internal space with the stage (20), and
the housing (10) is configured so that the internal space in a sealed state is formed by the stage (20) and the frame (30).

## Patentansprüche

1. System, umfassend:
einen Inkubator (200); und
eine Beobachtungsvorrichtung (500; 600), die in dem Inkubator (200) positioniert ist, wobei
die Beobachtungsvorrichtung (500; 600) enthält:
ein Gehäuse (10), das eine obere Platte enthält, die eine Bühne (20) ist, auf welcher ein Behälter (1), der eine Probe aufnimmt, positioniert ist;
ein optisches System (541; 641), das in dem Gehäuse (10) aufgenommen ist;
eine Bildgebungsvorrichtung (542; 644), die dazu ausgestaltet ist, ein Bild der Probe mit Licht von der Probe aufzunehmen, das durch die Bühne (20) und das optische System (541; 641) gelangt ist; und
einen Motor (64), der eine Leistungsquelle eines sich bewegenden Mechanismus ist, der dazu ausgestaltet ist, zu verursachen, dass sich das optische System (541; 641) in eine Richtung entlang der Bühne (20) bewegt, wobei
wenigstens entweder die Bildgebungsvorrichtung (542; 644) oder der Motor (64) außerhalb des Gehäuses (10) positioniert ist.

2. System nach Anspruch 1, die Beobachtungsvorrichtung ferner umfassend
eine optische Faser (642), die in einem optischen Gang zwischen dem optischen System (641) und der Bildgebungsvorrichtung (644), positioniert ist, die außerhalb des Gehäuses (10) positioniert ist, und die dazu ausgestaltet ist, das Licht von der Probe vom Inneren des Gehäuses (10) nach außerhalb des Gehäuses (10) zu leiten.

3. System nach Anspruch 1 oder 2, wobei
sowohl die Bildgebungsvorrichtung (644) als auch der Motor (64) außerhalb des Gehäuses (10) positioniert ist.

4. System nach einem der Ansprüche 1 bis 3, ferner umfassend
eine Lichtquellenvorrichtung (410), die außerhalb des Gehäuses (10) positioniert ist und dazu ausgestaltet ist, Beleuchtungslicht, das die Probe beleuchtet, auszusenden.

5. System nach Anspruch 4, wobei
die Lichtquellenvorrichtung (410) so ausgestaltet ist, dass sie an dem Behälter (1) frei befestigbar ist.

6. System nach einem der Ansprüche 1 bis 5, wobei
das Gehäuse (10) einen Rahmen (30) einschließt, der dazu ausgestaltet ist, einen Innenraum mit der Bühne (20) zu bilden, und
der Wärmewiderstand der Bühne (20) größer als der Wärmewiderstand des Rahmens (30) ist.

7. System nach Anspruch 6, wobei
die Bühne (20) wenigstens zwei flache Platten (21; 22) einschließt, die so positioniert sind, dass sich ein Vakuum zwischen den beiden flachen Platten befindet.

8. System nach Anspruch 6 oder Anspruch 7, wobei
die Bühne (20) eine flache Platte (22) einschließt, an welcher eine Oberflächenbehandlung zum Reflektieren eines Infrarotstrahls ausgeführt wurde.

9. System nach einem der Ansprüche 6 bis 8, wobei
das Gehäuse (10) den Rahmen (30) einschließt, an welchem eine Oberflächenbehandlung zur Förderung von Wärmeableitung nach außerhalb des Gehäuses (10) ausgeführt wurde.

10. System nach einem der Ansprüche 6 bis 9, wobei
der Rahmen (10) eine Struktur in Form eines Vorsprungs einschließt, die dazu ausgestaltet ist, Wärmeableitung nach außerhalb des Gehäuses (10) zu fördern.

11. System nach einem der Ansprüche 6 bis 10, wobei
das Gehäuse (10) ferner ein Wärmedämmelement (32) einschließt, das dazu ausgestaltet ist, Wärmeübergang von dem Rahmen (30) an die Bühne (20) zu verhindern.

12. System nach einem der Ansprüche 1 bis 11, ferner umfassend
eine Befestigungseinheit (80), die dazu ausgestaltet ist, die Beobachtungsvorrichtung (500; 600) an einer Innenwand des Inkubators (200) zu befestigen,
wobei
die Befestigungseinheit (80) einschließt:
ein Positionierungsgestell (81), auf welchem das Gehäuse (10) positioniert ist; und
wenigstens entweder ein erstes Wärmeleitelement (82) zwischen dem Positionierungsgestell (81) und der Innenwand (201), das dazu ausgestaltet ist, in engem Kontakt mit dem Positionierungsgestell (81) und der Innenwand (201) zu sein, oder ein zweites Wärmeleitelement (83) zwischen dem Positionierungsgestell (81) und dem Gehäuse (10), das dazu ausgestaltet ist, in engem Kontakt mit dem Positionierungsgestell (81) und dem Gehäuse (10) zu sein.

13. System nach einem der Ansprüche 1 bis 12, ferner umfassend
eine Wärmedämmeinheit (70), die so ausgestaltet ist, dass sie an einer oberen Oberfläche des Behälters (1) frei befestigbar ist und dass sie eine Abnahme einer Temperatur einer oberen Oberfläche des Behälters (1) unterdrückt.

14. System nach einem der Ansprüche 1 bis 13, wobei
das Gehäuse (10) einen Rahmen (30) einschließt, der dazu ausgestaltet ist, einen Innenraum mit der Bühne (20) zu bilden, und
das Gehäuse (10) so ausgestaltet ist, dass der Innenraum in einem abgedichteten Zustand von der Bühne (20) und dem Rahmen (30) gebildet ist.

## Revendications

1. Système comprenant :
un incubateur (200) ; et
un appareil d'observation (500 ; 600) placé dans l'incubateur (200),
l'appareil d'observation (500 ; 600) comprenant
un boîtier (10) comprenant une plaque supérieure qui est une platine (20) sur laquelle un récipient (1) qui contient un échantillon est placé ;
un système optique (541 ; 641) reçu dans le boîtier (10) ;
un dispositif d'imagerie (542 ; 644) configuré pour capturer une image de l'échantillon avec de la lumière de l'échantillon qui est entrée à travers la platine (20) et le système optique (541 ; 641) ; et
un moteur (64) qui est une source motrice d'un mécanisme de déplacement configuré pour amener le système optique (541 ; 641) à se déplacer dans une direction le long de la platine (20),
au moins un parmi le dispositif d'imagerie (542 ; 644) et le moteur (64) étant placé à l'extérieur du boîtier (10).

2. Système selon la revendication 1, l'appareil d'observation comprenant en outre
une fibre optique (642) placée dans un trajet optique entre le système optique (641) et le dispositif d'imagerie (644) placé à l'extérieur du boîtier (10), configurée pour guider la lumière de l'échantillon de l'intérieur du boîtier (10) à l'extérieur du boîtier (10).

3. Système selon la revendication 1 ou 2, dans lequel
à la fois le dispositif d'imagerie (644) et le moteur (64) sont placés à l'extérieur du boîtier (10).

4. Système selon l'une des revendications 1 à 3, comprenant en outre
un appareil de source de lumière (410) placé à l'extérieur du boîtier (10), configuré pour émettre une lumière d'éclairage qui éclaire l'échantillon.

5. Système selon la revendication 4, dans lequel
l'appareil de source de lumière (410) est configuré de façon à être apte à être fixé librement au récipient (1).

6. Système selon l'une des revendications 1 à 5, dans lequel
le boîtier (10) comprend un cadre (30) configuré pour former un espace interne avec la platine (20), et
une résistance thermique de la platine (20) est supérieure à une résistance thermique du cadre (30).

7. Système selon la revendication 6, dans lequel
la platine (20) comprend au moins deux plaques plates (21 ; 22) qui sont placées de telle sorte qu'un vide est pris en sandwich entre les deux plaques plates.

8. Système selon la revendication 6 ou la revendication 7, dans lequel
la platine (20) comprend une plaque plate (22) à laquelle un traitement de surface pour réfléchir un rayon infrarouge a été appliqué.

9. Système selon l'une des revendications 6 à 8, dans lequel
le boîtier (10) comprend le cadre (30) à lequel un traitement de surface pour favoriser la dissipation de chaleur vers l'extérieur du boîtier (10) a été appliqué.

10. Système selon l'une des revendications 6 à 9, dans lequel
le cadre (30) comprend une structure en forme de saillie, configurée pour favoriser la dissipation de chaleur vers l'extérieur du boîtier (10).

11. Système selon l'une des revendications 6 à 10, dans lequel
le boîtier (10) comprend en outre un élément d'isolation thermique (32) configuré pour bloquer un transfert de chaleur du cadre (30) à la platine (20).

12. Système selon l'une des revendications 1 à 11, comprenant en outre
une unité de fixation (80) configurée pour fixer l'appareil d'observation (500 ; 600) sur une paroi interne de l'incubateur (200),
l'unité de fixation (80) comprenant
un socle de placement (81) sur lequel le boîtier (10) est placé ; et
au moins un parmi un premier élément de conduction thermique (82) entre le socle de placement (81) et la paroi interne (201), configuré pour être en contact étroit avec le socle de placement (81) et la paroi interne (201), et un second élément de conduction thermique (83) entre le socle de placement (81) et le boîtier (10), configuré pour être en contact étroit avec le socle de placement (81) et le boîtier (10).

13. Système selon l'une des revendications 1 à 12, comprenant en outre
une unité d'isolation thermique (70) configurée de façon à être apte à être fixée librement sur une surface supérieure du récipient (1) et à supprimer une diminution d'une température d'une surface supérieure du récipient (1).

14. Système selon l'une des revendications 1 à 13, dans lequel
le boîtier (10) comprend un cadre (30) configuré pour former un espace interne avec la platine (20), et
le boîtier (10) est configuré de telle sorte que l'espace interne, dans un état scellé de manière étanche, est formé par la platine (20) et le cadre (30).
